# EUROPÄISCHE PATENTSCHRIFT

(11) **EP 3 139 871 B1**
(45) Veröffentlichungstag und Bekanntmachung des Hinweises auf die Patenterteilung: **17.07.2019**
(21) Anmeldenummer: 15732518.4
(22) Anmeldetag: 08.05.2015
(51) Int. Cl.: A61F 2/66, A61F 2/50

(54) **PROTHESENFUSS**
PROSTHETIC FOOT
PIED PROTHÉTIQUE

(30) Priorität: 09.05.2014 DE 102014006687
(43) Veröffentlichungstag der Anmeldung: 15.03.2017
(73) Patentinhaber: Ottobock SE & Co. KGaA, 37115 Duderstadt (DE)
(72) Erfinder: FRIESEN, Jeff, Salt Lake City, Utah 84121 (US); SMITH, Justin R., West Jordan, Utah 84081 (US); PIANYKH, Oleg, Salt Lake City, Utah 84121 (US)
(74) Vertreter: Lins, Martina
(86) Internationale Anmeldenummer: PCT/DE2015/100190
(87) Internationale Veröffentlichungsnummer: WO 2015/169291

(56) Entgegenhaltungen:
- EP-A1- 0 487 852
- EP-A1- 0 793 949
- DE-C1- 19 521 147
- US-B1- 6 811 571

## Beschreibung

Die Erfindung betrifft einen Prothesenfuß mit einem Kern, an dem eine proximale Befestigungseinrichtung zur Festlegung des Prothesenfußes an einem Unterschenkelrohr oder einem Unterschenkelschaft angeordnet ist und der von einer Fußhülle umgeben ist, die einen Hohlraum bildet, in dem der Kern eingesetzt ist.

Prothesenfüße sind die distalen Abschlüsse von Protheseneinrichtungen der unteren Extremität und werden entweder unmittelbar an einen Unterschenkelschaft oder aber an einem Unterschenkelrohr befestigt, das wiederum an einem Prothesenkniegelenk befestigt ist. Prothesenfüße können unterschiedlichste Ausgestaltungen aufweisen und Aufgaben erfüllen. Neben hoch komplexen, angetriebenen Prothesenfüßen, die ein Knöchelgelenk aufweisen, gibt es mehrteilige Federkonstruktionen, die den Nutzer dabei unterstützen sollen, ein möglichst dem natürlichen Gangverhalten angenähertes Gangbild zu ermöglichen. Vergleichsweise einfach aufgebaute Prothesenfüße sind die sogenannten SACH-Füße, die ein festes Knöchelgelenk und eine gepolsterte Ferse aufweisen. Ein solcher Prothesenfuß kann einem Holzkern aufweisen, der von einer Schaumstoffhülle umschäumt ist. Der Prothesenfuß bildet die äußere Form eines natürlichen Fußes nach, wobei es unterschiedliche Fußgrößen gibt, in die unterschiedlich große Kerne eingesetzt werden können.

Die US 8,128,709 B2 betrifft eine funktionale Fußumhüllung, bei der eine Fußhülle bereitgestellt ist, in die ein Prothesenfußeinsatz eingeführt ist. Der Prothesenfuß weist eine Basisfeder und ein gelenkig daran angeordnetes Knöchelelement auf, das an einem Unterschenkelrohr befestigbar ist. Die Prothesenfußhülle weist Zonen unterschiedlicher Elastizität im Sohlenbereich auf, um einerseits den Fersenstoß bei einem Auftreten des Prothesenfußes zu dämpfen und andererseits bei einem Abrollen über den Vorderfuß Energie zurückzugeben, um das Gangverhalten wie gewünscht zu beeinflussen. Die Materialeinsätze in der Fußhülle können sowohl an der Außenseite als auch an der Innenseite angeordnet oder eingeformt sein. Das Abrollverhalten wird über die Basisfeder des Prothesenfußeinsatzes maßgeblich bestimmt.

Die EP 0 487 852 A1 betrifft einen gelenklosen Prothesenfuß mit einem innerhalb des Prothesenkörpers vorgesehenen, die Prothesenbelastungen aufnehmenden federelastischen Fußeinsatz, an dessen oberen horizontalen Schenkel ein Fußadapter angeordnet ist. Ein unterer Schenkel ist gegenüber dem oberen Schenkel verlängert ausgebildet und erstreckt sich mit seinem freien Ende bis in den Fußspitzenbereich des Prothesenfußes. Der Fußeinsatz weist eine angenähert S-förmige Ausbildung auf. Der Fußeinsatz ist innerhalb eines Prothesenkörpers angeordnet, der mit Ausnahme der oberen Anschlussfläche vollständig von einer hautbildenden äußeren Kunststoffschaumschicht ummantelt ist, die einen sich bis in den Zehenbereich erstreckenden Innenfuß aus Kunststoffschaum umschließt.

Die US 2005/0071018 A1 betrifft einen Prothesenfuß mit einem Knöchelblock, an dessen oberen Ende Befestigungselemente zur Anbringung des Prothesenfußes an einem Unterschenkelrohr befestigt sind. An der Unterseite des Knöchelblocks ist eine Sohlenplatte aus einem stabilen, flexiblen Material befestigt. Die Sohlenplatte und der Knöchelblock sind von einer Fußkosmetik umgeben, die an dem vorderen und hinteren Bereich der Sohlenplatte angeordnet ist. Im Zehenbereich ist ein Verstärkungselement angeordnet.

Ein Prothesenfuß gemäß des Oberbegriffes von Anspruch 1 ist aus der US 6,811,571 B1 bekannt.

Aufgabe der vorliegenden Erfindung ist es, einen Prothesenfuß bereitzustellen, der preiswert und modular herzustellen ist und über den eine Anpassung der Fußeigenschaften kostengünstig erfolgen kann.

Erfindungsgemäß wird diese Aufgabe durch einen Prothesenfuß mit den Merkmalen des Hauptanspruches gelöst. Vorteilhafte Ausgestaltungen und Weiterbildungen der Erfindung sind in den Unteransprüchen, der Beschreibung und den Figuren offenbart.

Der Prothesenfuß mit einem Kern, an dem eine proximale Befestigungseinrichtung zur Festlegung des Prothesenfußes an einem Unterschenkelrohr oder an einem Unterschenkelschaft angeordnet ist und der von einer Fußhülle umgeben ist, die einen Hohlraum ausgebildet, in dem der Kern eingesetzt ist, sieht vor, dass in einem Vorderfußbereich der Fußhülle ein separater Aufnahmeabschnitt mit einer schlitzförmigen Ausnehmung ausgebildet ist, in die eine an dem Kern angeordnete Zunge eingeführt ist. Der Kern weist eine Blattfeder auf, dessen vorderes Ende die Zunge ausbildet. Alternativ ist es vorgesehen, dass an dem Kern die Zunge befestigt ist, wobei die Zunge über das vordere Ende des Kerns hinausragt. Die Zunge ist somit als separates Bauteil an dem Kern befestigt. Es ist auch möglich, dass die Blattfeder den Kern bildet, so dass an der Blattfeder als Kern die Zunge als separates Bauteil befestigt sein kann. Durch die Ausgestaltung des Kerns als Blattfeder oder die Ausstattung des Kerns mit einer Blattfeder ist es möglich, das Abrollverhalten weiter zu differenzieren und eine zusätzliche Elastizität, die über die Elastizität der Fußhülle hinausgeht, bereitzustellen. Dadurch kann eine verbesserte Anpassung an das natürliche Abrollverhalten erreicht werden.

Durch die formschlüssige Festlegung des Kernes über den separaten Aufnahmeabschnitt mit der darin eingeführten Zunge in dem Vorderfußbereich ist es möglich, die Fußfunktion so zu beeinflussen, wie es gewünscht ist. Zur Fußfunktion zählen im Wesentlichen das Fersenverhalten, das Roll-Over-Verhalten und das Vorfußverhalten. Das Fersenverhalten und das Roll-Over-Verhalten bis zur mittleren Standphase werden durch das Verformungsverhalten des Kerns, sofern dieser verformbar ist, sowie einer gegebenenfalls vorhandenen Fersenfeder oder durch das elastische Verhalten der Fußhülle bestimmt. Das Vorfußverhalten und das Roll-Over-Verhalten ab der mittleren Standphase werden wiederum durch den Kern und die elastischen Eigenschaften der Fußhülle im Vorderfußbereich definiert. Über die formschlüssige Verbindung des vorderen Teils des Kerns, der in Gestalt einer Zunge ausgebildet ist, einen zungenförmigen Vorsprung aufweist oder an dem eine Zunge befestigt ist, ist es möglich, durch eine Variation entweder der Fußhülle, des Kernes, des Aufnahmeabschnitts oder der Zunge die Eigenschaften, insbesondere die effektive Fußlänge zu beeinflussen. Die effektive Fußlänge ist dabei der Abstand der Kraftangriffspunkte beim Überrollen, während sich der Prothesenfuß sowohl mit der Ferse als auch mit dem Vorderfuß auf dem Untergrund befindet. Die effektive Fußlänge wird durch die Steifigkeit im Vorderfußbereich verändert. Ist der Vorderfußbereich weicher, verhält sich der Prothesenfuß wie ein kürzerer Prothesenfuß mit einem starren Vorderfußbereich, wird ein härterer oder steiferer Vorderfußbereich eingestellt, vergrößert sich die effektive Fußlänge, der Prothesenfuß verhält sich somit wie ein vergleichsweise längerer Prothesenfuß. In dem Schlitz in dem Aufnahmeabschnitt ist die Zunge eingeführt, so dass der Vorderfußbereich eine abweichende Steifigkeit gegenüber der übrigen Fußhülle aufweisen kann, in der die Zunge oder der Aufnahmeabschnitt nicht eingeführt ist.

Der Aufnahmeabschnitt ermöglicht eine einstellbare Härte des Vorfußes, ohne dass eine Veränderung der gesamten Fußhüllenform oder der Materialzusammensetzung der Füßhülle notwendig wird. Die Ausgestaltung des Aufnahmeabschnittes als separates Element bewirkt eine stoffliche Trennung von Fußhülle und Kern, so dass die Spannungen in dem Bereich des Fußballens reduziert werden. Die Fußhülle und der Aufnahmeabschnitt können aus einem Schaumwerkstoff ausgebildet sein. Durch die Trennung der beiden Schaumwerkstoffe, die insbesondere formschlüssig miteinander gekoppelt sind, verringert sich die mechanische Belastung und die Haltbarkeit der Fußhülle und des Aufnahmeabschnittes wird erhöht. Zudem ist es möglich, unterschiedliche Fußgrößen mit nur einem Fußmodul oder Kern und unterschiedlichen Fußhüllen zu realisieren, indem der Aufnahmeabschnitt ausgewechselt und an die jeweilige Fußgröße angepasst wird.

Die separate Ausgestaltung von Fußhülle und Aufnahmeabschnitt ermöglicht es, dass unterschiedliche Härten des Vorfußes eingestellt werden können, indem unterschiedliche Materialien verwendet werden. Hieraus ergibt sich eine Gewichtsreduzierung, da die Fußhülle und der Aufnahmeabschnitt getrennt voneinander hergestellt und funktionsangepasst ausgewählt werden können. Die Fußhülle muss in der Regel eine größere mechanische Stabilität gegen Reibung oder Verschleiß als der Aufnahmeabschnitt aufweisen, der Aufnahmeabschnitt muss dagegen hinsichtlich der gewünschten Härte oder Deformierbarkeit leicht einstellbar sein. Die für die jeweiligen Anforderungen optimalen Materialien sind in der Regel verschiedenen und können hinsichtlich ihrer Dichten optimiert ausgewählt werden, so dass sich eine Gewichtsreduktion im Vergleich zu einer einteiligen Fußhülle oder einem einteiligen Kern ergibt.

Der Aufnahmeabschnitt vergrößert die Auflagefläche des Kerns innerhalb der Fußhülle, so dass dadurch Spannungsspitzen reduziert und die Haltbarkeit der Fußhülle vergrößert werden.

Gurte oder starre Materialien wie Kunststoff- oder Metallplatten können in der Fußhülle oder dem Aufnahmeabschnitt angeordnet, insbesondere eingeschäumt werden, um eine ggf. gewünschte Verlängerung des Vorfußes beim Abstoßen zu ermöglichen.

Die Ausnehmung zur Aufnahme der Zunge ist in dem Aufnahmeabschnitt ausgebildet, der wiederum den Vorderfußbereich der Fußhülle ausfüllend ausgebildet sein kann. Der Aufnahmeabschnitt und damit der Vorderfußbereich der Fußhülle sind somit vollständig mit Material ausgefüllt, in das ein Schlitz eingebracht oder eingeformt ist. Über dem Aufnahmeabschnitt wird eine ausreichende mechanische Festigkeit der Fußhülle erreicht, so dass Kräfte von der Zunge über den Aufnahmeabschnitt auf die Fußhülle übertragen werden können, wobei die Fußhülle den äußeren Abschluss des Prothesenfußes bildet und eine dem natürlichen Fuß angenäherte Formgebung aufweist. Die Fußhülle ist vorteilhafterweise aus einem elastischen Kunststoffmaterial, insbesondere einem Schaummaterial ausgebildet, der eine ausreichende Festigkeit zur Aufnahme und Übertragung der Kräfte, einen ausreichenden Verschleißwiderstand sowie die gewünschten elastischen und optischen Eigenschaften aufweist. Der Vorderfußbereich der Fußhülle ist somit massiv ausgebildet und nimmt die Zunge des Kerns auf.

Der Vorderfußbereich und/oder Aufnahmeabschnitt kann entweder aus dem gleichen Material bestehen, aus dem die übrige Fußhülle besteht, ebenso ist es möglich, dass der Vorderfußbereich und/oder der Aufnahmeabschnitt eine von der übrigen Fußhülle abweichende Steifigkeit aufweist, wodurch es möglich ist, eine Anpassung der Vorderfußsteifigkeit und des elastischen Verhaltens der Fußhülle an den Patienten oder die gewünschten Eigenschaften des Prothesenfußes zu erreichen. Dadurch ist es möglich, dass Fußhüllen gleicher Größe mit baugleichen Kernen bestückt werden und unterschiedliche Abrollverhalten aufweisen, indem z.B. die Aufnahmeabschnitte mit unterschiedlichen Steifigkeiten ausgestattet werden. Der Aufnahmeabschnitt kann in der Fußhülle eingesetzt und dort durch Haftkräfte, Klebekräfte oder Formschlusselemente gehalten sein. Bevorzugt ist der Aufnahmeabschnitt austauschbar oder aus der Fußhülle herausnehmbar ausgebildet, um eine leichte Anpassbarkeit an die jeweiligen Anforderungen vornehmen zu können. Ebenso ist es möglich, dass der Aufnahmeabschnitt in der Fußhülle eingeformt oder daran angeformt ist, beispielsweise im Rahmen des Urformverfahrens beim Gießen oder Spritzgießen der Fußhülle. Ergänzend oder alternativ zu dem Einsetzen ist es möglich, dass der Aufnahmeabschnitt in der Fußhülle verklebt ist. Es wird zunächst der Aufnahmeabschnitt aus einem Material mit den gewünschten elastischen und mechanischen Eigenschaften gefertigt und anschließend die übrige Fußhülle mit dem Hohlraum zur Aufnahme des Kerns daran angeformt oder umgekehrt der Aufnahmeabschnitt in dem Hohlraum eingegossen.

Vorteilhafterweise ist der Kern in der Fußhülle auswechselbar eingesetzt und herausnehmbar ausgebildet. Bevorzugt wird der Kern rein formschlüssig in der Fußhülle und/oder dem Aufnahmeabschnitt gehalten, unterstützt von den Haftkräften, die aufgrund der Materialeigenschaften der Fußhülle bzw. des Aufnahmeabschnittes auftreten. Insbesondere im Fersenbereich sind dazu Formschlusselemente an dem Kern und korrespondierende Formschlusselemente an der Fußhülle vorgesehen, beispielsweise in Form von Nuten und Vorsprüngen oder Hinterschnitten, in die korrespondierende Formschlusselemente der jeweils anderen Komponente eingreifen, so dass eine auswechselbare und formschlüssige Festlegung der Fußhülle an dem Kern leicht erfolgen kann. Gleiches kann auch für den Aufnahmeabschnitt in der Fußhülle und der Zunge in dem Aufnahmeabschnitt ausgebildet sein.

Der Kern kann ein Fersenpolster aufweisen, das entweder aus einem Schaumstoff besteht, das neben elastischen Eigenschaften auch eine Dämpfung bereitstellt. Auch ist es möglich, dass eine Fersenfeder vorgesehen ist, deren Dämpfungsverhalten in der Regel verschieden von dem Dämpfungsverhalten eines Schaumkörpers ist.

Die Zunge kann als Gurt oder Blattfeder ausgebildet sein, wobei als Gurtwerkstoff ein hochfestes Gewebe mit einer ausreichenden Steifigkeit vorgesehen sein kann. Es besteht die Möglichkeit, die Zunge in den Aufnahmeabschnitt einzuformen und nachträglich an dem Kern festzulegen, wenn dieser in den Hohlraum eingeführt wird. Ebenso ist es möglich, die Zunge zusammen mit dem separat ausgebildeten Aufnahmeabschnitt beim Einführen zusammen mit dem Kern in der Fußhülle zu positionieren und dort festzulegen.

Ein Gurt oder eine Blattfeder kann in den Aufnahmeabschnitt und/oder in die Fußhülle integriert sein, um eine ergänzende Einstellmöglichkeit hinsichtlich der Steifigkeit der Fußhülle oder Fußkosmetik bereitzustellen.

Die Zunge ist vorteilhafterweise an der Unterseite des Kerns befestigt, also an derjenigen Seite, die der Sohle oder dem Untergrund zugewandt ist. Um eine maximale Beeinflussung des Abrollverhaltens zu erreichen, ist vorteilhafterweise vorgesehen, dass die Zunge bis in den Zehenbereich hineinragt, wobei es vorgesehen sein kann, dass die Zunge eine sich über die Länge verändernde Breite aufweist, um eine Anpassung an die Fußform und die geometrischen Gegebenheiten zu erreichen. Soll die Zunge beispielsweise nur bis in den vorderen Großzehenbereich hineinragen, ist es notwendig, die Breite zu verringern. Grundsätzlich ist es auch möglich, die Zunge mehrteilig auszubilden oder sich aufspreizen zu lassen, so dass ein Teil der Zunge sich in den Großzehenbereich und ein anderer Teil sich in dem übrigen Zehenbereich erstreckt.

Die Fußhülle oder Fußkosmetik kann in dem Mittelfußbereich eine Versteifung auch zusätzlich zu der Zunge aufweisen, die sich in dem Vorderfußbereich erstreckt, um die jeweils gewünschte Steifigkeit der Fußhülle und damit auch des gesamten Prothesenfußes wunschgemäß einstellen zu können.

Eine Variante der Erfindung sieht vor, dass in dem Vorderfußbereich, beispielsweise in der Fußhülle oder aber in einem Aufnahmeabschnitt, ein separates Einlegeelement angeordnet ist, das eine von der Fußhülle oder dem Aufnahmeabschnitt abweichende Steifigkeit aufweist, um die Fußhülle als funktionales Bauteil ausbilden zu können. Durch das Einlegeelement ist es möglich, die Fußhülle selbst in der Steifigkeit anzupassen, beispielsweise indem das Einlegeelement auswechselbar innerhalb der Fußhülle oder dem Aufnahmeabschnitt befestigt ist. Das Aufnahmeelement kann in dem Aufnahmeabschnitt oder in der Hülle eingelegt, eingeklebt oder für einen dauerhaft Verbleib in der Fußhülle, dem Aufnahmeabschnitt, auf jeden Fall aber im Vorderfußbereich eingelegt, eingeklebt oder eingeformt werden.

Die Versteifung ist vorteilhafterweise so ausgebildet, dass sich während der terminalen Standphase im Abrollvorgang der Krafteinleitungspunkt nach anterior verschiebt, um die effektive Fußlänge zu vergrößern.

Neben einer Ausgestaltung des Prothesenfußes mit einem separaten Aufnahmeabschnitt kann der Vorderfußbereich der Fußhülle eine schlitzförmige Ausnehmung aufweisen, in der eine an dem Kern angeordnete Zunge eingeführt ist, wobei der Vorderfußbereich eine im Vergleich zur übrigen Fußhülle abweichende Steifigkeit aufweist. Der Vorderfußbereich kann massiv ausgebildet sein.

Nachfolgend werden Ausführungsbeispiele der Erfindung anhand der beigefügten Figuren näher erläutert. Es zeigen:
- Figur 1: eine schematische Querschnittsansicht eines Prothesenfußes;
- Figur 2: eine Variante mit einem veränderten Aufnahmeabschnitt;
- Figur 3: eine Variante mit separater Zunge;
- Figur 4: eine Variante gemäß Figur 2 mit einer separaten Zunge;
- Figur 5: eine Variante der Figur 1 mit einem separaten Einlegeelement; sowie
- Figur 6: eine Variante der Figur 5.

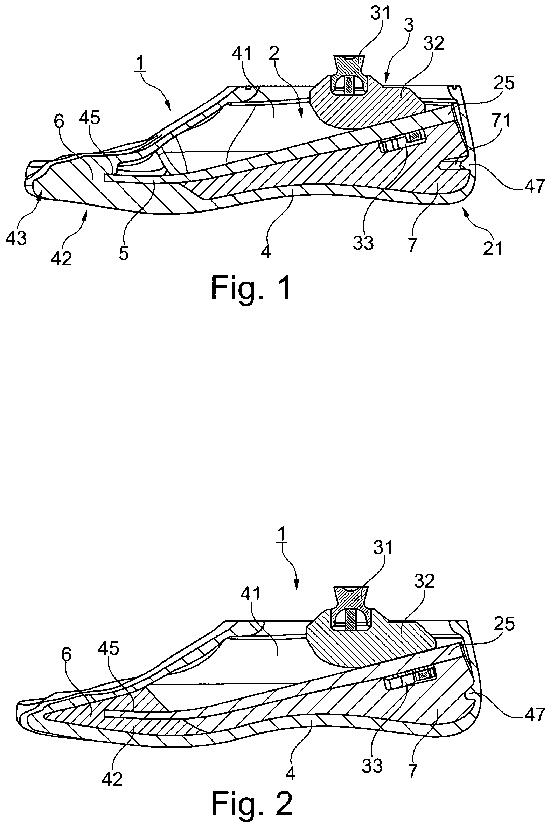

In der Figur 1 ist in einer schematischen Schnittdarstellung ein Prothesenfuß 1 mit einem Kern 2 dargestellt, der eine proximale Befestigungseinrichtung 3 mit einem Schraubpylon 31 und einem Befestigungsadapter 32 über Schrauben 33 befestigt ist. Der Befestigungsadapter 32 ist auswechselbar an dem Kern 2 befestigt, um eine Anpassbarkeit an die unterschiedlichen Nutzer, Absatzhöhen, Schuhformen oder dergleichen zu ermöglichen. Der Kern 2 weist eine Blattfeder 25 auf, an dessen Oberseite die Befestigungseinrichtung 3 befestigt ist. An der Unterseite der Blattfeder 25, also an derjenigen Seite, die zur Sohle oder zum Boden gerichtet ist, ist ein Fersenpolster 7 aus einem Schaummaterial angeordnet. Das Fersenpolster 7 ist im dargestellten Ausführungsbeispiel mit der aus einem faserverstärkten Kunststoffmaterial, beispielsweise einem carbonfaserverstärkten Kunststoff, bestehenden Blattfeder verklebt. Die Oberseite des Fersenpolsters 7 ist vollflächig mit der Unterseite der Blattfeder 25 verklebt, Aussparungen für die Schraube 33 oder die Schrauben 33 zur Befestigung des Befestigungsadapters 32 sind vorgesehen. Im Fersenbereich 21 des Prothesenfußes 1 ist im Fersenpolsters 7 ist eine Nut 71 ausgebildet, in die ein Vorsprung 47, der an der Innenseite der Fußhülle 4 ausgebildet ist, eingreift und den Fersenbereich der Fußhülle 4 formschlüssig an dem Fersenbereich 21 des Kerns 2 festlegt.

Die Fußhülle 4, die aus einem Schaumwerkstoff ausgebildet ist, bildet einen Hohlraum 41 aus, in dem der Kern 2, der im dargestellten Ausführungsbeispiel aus der Blattfeder 25 und dem Fersenpolster 7 besteht, eingeführt ist. Im Vorderfußbereich 42 der Fußhülle 4 ist ein Aufnahmeabschnitt 6 vorhanden, in dem eine schlitzförmige Ausnehmung 45 ausgebildet ist. In diese schlitzförmige Ausnehmung 45 greift das vordere Ende der Blattfeder 25 ein, die als Zunge 5 ausgebildet ist und über das vordere Ende des Fersenpolsters 5 hinaussteht. Die Zunge reicht vom Bereich des Zehengrundgelenkes bis in den mittleren Zehenbereich 43 hinein und hält den Kern 2 formschlüssig im Vorderfußbereich 42 der Fußhülle 4 fest. Der Aufnahmeabschnitt 6 ist vollständig mit dem Werkstoff der übrigen Fußhülle 4 ausgefüllt und stellt eine ausreichende mechanische Stabilität bereit, um bei einer Vorderfußbelastung Kräfte von dem Prothesenfußnutzer auf dem Boden zu übertragen. Durch die formschlüssige und auswechselbare Ausgestaltung der Befestigung des Kernes 2 innerhalb der Fußhülle 4 über die Zunge 5 und die schlitzförmige Ausnehmung 45 ist es möglich, bei einer Verformung der elastischen Fußhülle 4 und der Blattfeder 25 während des Abrollvorganges eine Relativbewegung zuzulassen, so dass nur eine geringe Scherbelastung innerhalb der Fußhülle 4 erzeugt wird.

Figur 2 zeigt eine Variante der Erfindung bei der statt einer einstückigen Ausgestaltung der Fußhülle 4 mit dem Aufnahmeabschnitt 6 ein separater Aufnahmeabschnitt 6 innerhalb der Fußhülle 4 angeordnet ist. Der Aufnahmeabschnitt 6 kann an der übrigen Fußhülle 4 angeformt, angeklebt oder formschlüssig festgehalten sein. Der Aufnahmeabschnitt 6 weist eine Steifigkeit und ein Elastizitätsverhalten auf, das sich von dem Verhalten der übrigen Fußhülle 4 unterscheidet, so dass durch den separaten Aufnahmeabschnitt 6 oder den ein- oder angeformten Aufnahmeabschnitt 6 aus einem anderen Material eine Veränderung des elastischen Verhaltens des Prothesenfußes 1 erzielen lässt. Der Aufnahmeabschnitt 6 erstreckt sich bis in den vorderen Zehenbereich 43 hinein, so dass die gesamte Länge des Prothesenfußes 1 ausgenutzt werden kann, um das Gangverhalten zu beeinflussen. Je steifer der Aufnahmeabschnitt 6 ausgebildet ist, desto größer ist die effektive Fußlänge, je weicher der Aufnahmeabschnitt 6 ausgebildet ist, desto kürzer ist die effektive Fußlänge. Der separate Aufnahmeabschnitt 6 kann zusammen mit dem Kern 2 in den Hohlraum 41 der Fußhülle 4 bei der Endmontage des Prothesenfußes 1 eingeführt werden. Ebenso ist es möglich, dass der separate Aufnahmeabschnitt 6 zunächst in die Fußhülle 4 eingeführt und dort formschlüssig, kraftschlüssig oder stoffschlüssig festgelegt wird. Anschließend wird die Zunge 5 als vorderer Teil der Blattfeder 25 in die Ausnehmung 45 eingeführt, um eine mechanische Verriegelung der Zunge 5 und damit des Kerns 2 in dem Hohlraum 41 der Fußhülle 1 zusammen mit der formschlüssigen Verbindung im Fersenbereich 21 über die Nut 71 und den Vorsprung 47 zu bewirken.

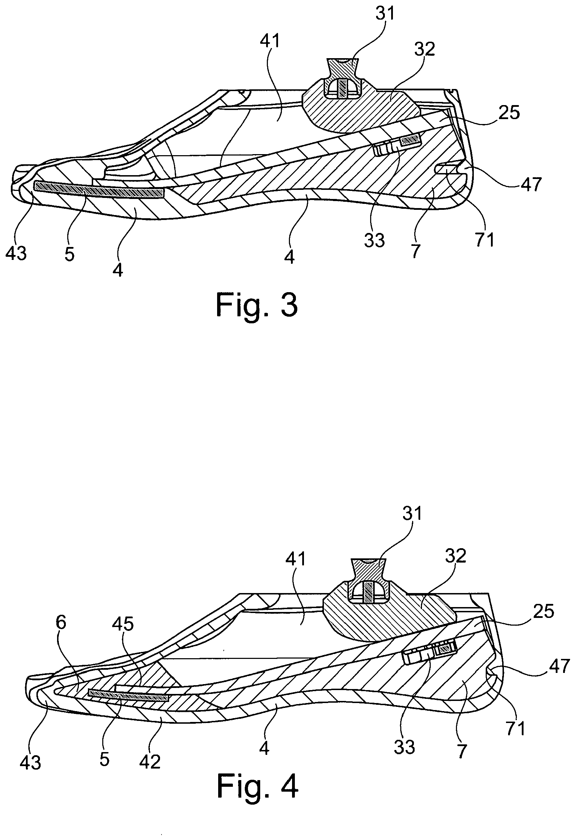

Ein weiteres Beispiel ist in der Figur 3 dargestellt, bei der der Aufbau grundsätzlich dem der Figur 1 entspricht, so dass hierauf vollinhaltlich Bezug genommen wird. An der Unterseite der Blattfeder 25 ist im Vorderfußbereich 42 eine separate Zunge 5 angeordnet, die bis in den vorderen Zehenbereich 43 der Fußhülle 4 hineinragt. Die separate Zunge 5 kann als Blattfeder mit abweichender Federsteifigkeit im Verhältnis zu der Blattfeder 25 des Kerns 2 ausgebildet sein, ebenso ist es möglich, dass die Zunge 5 als Gurt, Band oder anderes Einlegeteil ausgebildet ist, das an der Unterseite der Blattfeder 25 angeordnet und dort festgelegt ist. Die Festlegung kann beispielsweise durch Verkleben, Verschweißen oder durch mechanische Befestigungsmittel wie Schrauben, Niete, Clipse oder dergleichen erfolgen. Die Ausbildung mit einer separaten Zunge hat den Vorteil, dass die Variabilität bei der Ausgestaltung und Einstellung des Prothesenfußes einfach und kostengünstig vergrößert werden kann.

Die Figur 4 zeigt die Variante gemäß Figur 2 mit einer separaten Zunge 5. Die kraftübertragende Verbindung zwischen den separaten Zungen 5 und dem jeweiligen Kern 2 oder der Blattfeder 25 ermöglicht eine Einstellung der effektiven Fußlänge. Die Verlängerung der effektiven Fußlänge kann dabei bis in den vorderen Zehenbereich erfolgen, so dass eine maximale effektive Fußlänge erreicht werden kann.

In den Ausführungsbeispielen ist der Kern 2 als Federelement 25 aus einem Kohlefaser-Verbundbauteil mit einem Fersenpolster 7 ausgebildet. Das Fersenpolster 7 beeinflusst maßgeblich die Eigenschaften des Prothesenfußes 1 von einem Fersenauftritt bis zur mittleren Standphase. Das Fersenpolster 7 erstreckt sich bis ca. 2/3 der gesamten Fußlänge von der Ferse in Richtung auf den Vorderfuß. Die Länge des Fersenpolsters 7 entspricht ungefähr 75% der Länge der Blattfeder 25. Die Befestigungseinrichtung 3 und der Kern 2 aus Blattfeder 25 und Fersenpolster 7 ergeben ein Fußmodul, das seinerseits in einen korrespondierend ausgebildeten Hohlraum 41 einer Fußhülle eingeführt ist und über Formschlusselemente 71, 47, 45, 5 darin gehalten ist. Die Verbindung zwischen der Fußhülle 4 und dem Fußmodul ist nicht permanent, vielmehr ist die Fußhülle 4 lösbar an dem Fußmodul befestigt. Die Fußhülle 4 kann aus einem Schaummaterial bestehen oder auch verschiedene Schaummaterialien aufweisen. Die Schaummaterialien können unterschiedliche Dichten und Härten aufweisen, ebenfalls ist es möglich, dass nur bereichsweise unterschiedliche Schaummaterialien oder gleiche Schaummaterialien unterschiedlicher Härte und Dichte aufweisen.

Durch das Steuern der Härte der Fußhülle 4 oder des Aufnahmeabschnitts 6 im Vorderfußbereich kann die effektive Fußlänge beeinflusst werden.

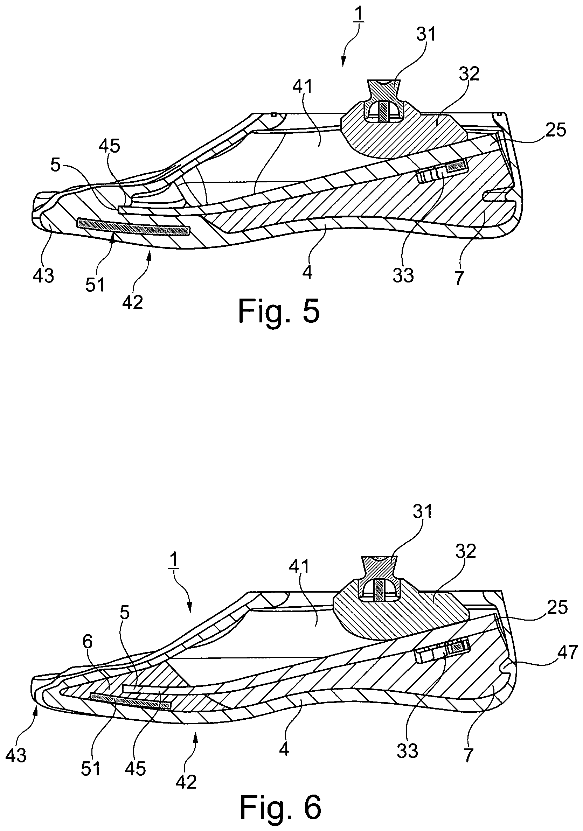

In der Figur 5 ist eine schematische Darstellung einer beispielhaften Variante der Figur 1 dargestellt, bei der im Vorderfußbereich 42 ein separates Einlegeelement 51, das sich bis zum Zehenbereich 43 erstreckt, eingelegt ist. Das Einlegeelement 51 kann als Blattfeder, Gurt oder Schaumstoffelement mit einer Festigkeit ausgebildet sein, die sich von der Festigkeit des Werkstoffes im Vorderfußbereich 42, insbesondere im ausfüllten vorderen Vorderfußbereich 42 unterscheidet, um so in einem Zusammenwirken mit der Blattfeder 25 die effektive Fußlänge beeinflussen zu können. Im dargestellten Ausführungsbeispiel ist das Einlegeelement 51 eingeschäumt, alternativ kann es auswechselbar befestigt eingeschoben oder eingeklebt werden.

Eine weitere Variante ist in der Figur 6 gezeigt, wobei das separate Einlegeelement 51 auf der Unterseite des Aufnahmeabschnittes 6 angeordnet ist. Das Einlegeelement 51 kann auswechselbar in einer Ausnehmung innerhalb des Aufnahmeabschnittes 6 eingelegt oder eingeklebt sein und anschließend von der Fußhülle abgedeckt und sicher an dem Aufnahmeabschnitt 6 gehalten sein. Ebenso ist es möglich, dass das Einlegeelement 51 angeformt oder eingeformt ist, wobei die Materialien und damit auch die Steifigkeit des Einlegeelementes 51 sich danach richten, welche elastischen Eigenschaften im Vorderfußbereich 42 erzielt werden sollen und welche effektive Fußlänge über das Einlegeelement 51 eingestellt werden soll. Das Einlegeelement 51 ragt über die vordere Spitze der Zunge 5 und damit auch über die vordere Spitze der Blattfeder 25 hinaus. Es ist auch möglich, dass das separate Einlegeelement 51 in Verbindung mit einer separaten Zunge 5, wie sie in den Figuren 3 und 4 gezeigt ist, zum Einsatz gelangt.

## Patentansprüche

1. Prothesenfuß mit einem Kern (2), an dem eine proximale Befestigungseinrichtung (3) zur Festlegung des Prothesenfußes (1) an einem Unterschenkelrohr oder einem Unterschenkelschaft angeordnet ist und der von einer Fußhülle (4) umgeben ist, die einen Hohlraum (41) ausbildet, in dem der Kern (2) eingesetzt ist, wobei in einem Vorderfußbereich (42) der Fußhülle (4) ein Aufnahmeabschnitt (6) mit einer schlitzförmigen Ausnehmung (45) ausgebildet ist, in die eine an dem Kern (2) angeordnete Zunge (5) eingeführt ist, wobei der Kern (2) eine Blattfeder (25) aufweist oder als Blattfeder ausgebildet ist und dessen vorderes Ende die Zunge (5) ausbildet und/oder die Zunge (5) an dem Kern (2) befestigt ist und über das vordere Ende des Kerns (2) hinausragt, **dadurch gekennzeichnet, dass** der Aufnahmeabschnitt (6) als separater Aufnahmeabschnitt ausgebildet ist.

2. Prothesenfuß nach Anspruch 1, **dadurch gekennzeichnet, dass** der Aufnahmeabschnitt (6) den Vorderfußbereich (42) ausfüllt.

3. Prothesenfuß nach Anspruch 2, **dadurch gekennzeichnet**, das der Vorderfußbereich (42) und/oder der Aufnahmeabschnitt (6) eine von der übrigen Fußhülle (4) abweichende Steifigkeit aufweist.

4. Prothesenfuß nach Anspruch 2 oder 3, **dadurch gekennzeichnet, dass** der Aufnahmeabschnitt (6) in der Fußhülle (4) eingesetzt, und/oder verklebt oder angeformt ist.

5. Prothesenfuß nach einem der voranstehenden Ansprüche, **dadurch gekennzeichnet, dass** der Kern (2) in einem Fersenbereich (21) formschlüssig an der Fußhülle (4) festgelegt ist.

6. Prothesenfuß nach einem der voranstehenden Ansprüche, **dadurch gekennzeichnet, dass** der Kern (2) ein Fersenpolster (7) aufweist.

7. Prothesenfuß nach einem der voranstehenden Ansprüche, **dadurch gekennzeichnet, dass** die Zunge (5) als Gurt oder Blattfeder ausgebildet ist.

8. Prothesenfuß nach einem der voranstehenden Ansprüche, **dadurch gekennzeichnet, dass** ein Gurt oder eine Blattfeder in den Aufnahmeabschnitt (6) und/oder in die Fußhülle (4) integriert ist.

9. Prothesenfuß nach einem der voranstehenden Ansprüche, **dadurch gekennzeichnet, dass** die Zunge (5) an der Unterseite des Kerns (2) befestigt ist.

10. Prothesenfuß nach einem der voranstehenden Ansprüche, **dadurch gekennzeichnet, dass** die Zunge (5) bis in einen Zehenbereich (43) des Prothesenfußes (1) hineinragt.

11. Prothesenfuß nach einem der voranstehenden Ansprüche, **dadurch gekennzeichnet, dass** in dem Vorderfußbereich (42) ein separates Einlegeelement (51) mit einer von der Fußhülle (4) oder dem Aufnahmeabschnitt (6) abweichenden Steifigkeit angeordnet ist.

12. Prothesenfuß nach einem der voranstehenden Ansprüche, **dadurch gekennzeichnet, dass** durch eine Versteifung im Vorderfußbereich (42) der Krafteinleitungspunkt während der terminalen Standphase nach anterior verschoben ist.

13. Prothesenfuß nach einem der voranstehenden Ansprüche, **dadurch gekennzeichnet, dass** die Fußhülle (4) in dem Mittelfußbereich eine Versteifung aufweist, die sich bis in den Vorderfußbereich (42) erstreckt.

14. Prothesenfuß nach einem der voranstehenden Ansprüche, **dadurch gekennzeichnet, dass** der Aufnahmeabschnitt (6) und/oder der Kern (2) austauschbar oder aus der Fußhülle (4) herausnehmbar ausgebildet ist.

## Claims

1. A prosthetic foot comprising a core (2), on which a proximal fastening device (3) for securing the prosthetic foot (1) to a below knee tube or a below knee shank is arranged and which is surrounded by a foot casing (4) which forms a cavity (41) into which the core (2) has been inserted, wherein a receiving portion (6) with a slot-shaped recess (45) is formed in a forefoot region (42) of the foot casing (4), with a tongue (5) arranged on the core (2) being inserted into said recess (45), wherein the core (2) has a leaf spring (25) or is embodied as a leaf spring and the front end of thereof forms the tongue (5) and/or the tongue (5) is fastened to the core (2) and projects beyond the front end of the core (2), **characterized in that** the receiving portion (6) is embodied as a separate receiving portion.

2. The prosthetic foot as claimed in claim 1, **characterized in that** the receiving portion (6) fills the forefoot region (42).

3. The prosthetic foot as claimed in claim 2, **characterized in that** the forefoot region (42) and/or the receiving portion (6) has/have a rigidity deviating from that of the remaining foot casing (4).

4. The prosthetic foot as claimed in claim 2 or 3, **characterized in that** the receiving portion (6) is inserted into the foot casing (4) and/or adhesively bonded thereto or formed thereon.

5. The prosthetic foot as claimed in one of the preceding claims, **characterized in that** the core (2) is secured with form fit to the foot casing (4) in a heel region (21).

6. The prosthetic foot as claimed in one of the preceding claims, **characterized in that** the core (2) has a heel cushioning (7).

7. The prosthetic foot as claimed in one of the preceding claims, **characterized in that** the tongue (5) is embodied as a belt or leaf spring.

8. The prosthetic foot as claimed in one of the preceding claims, **characterized in that** a belt or a leaf spring is integrated into the receiving portion (6) and/or into the foot casing (4).

9. The prosthetic foot as claimed in one of the preceding claims, **characterized in that** the tongue (5) is fastened to the lower side of the core (2).

10. The prosthetic foot as claimed in one of the preceding claims, **characterized in that** the tongue (5) projects into a toe region (43) of the prosthetic foot (1).

11. The prosthetic foot as claimed in one of the preceding claims, **characterized in that** a separate insertion element (51) with a rigidity deviating from the foot casing (4) or the receiving portion (6) is arranged in the forefoot region (42).

12. The prosthetic foot as claimed in one of the preceding claims, **characterized in that** the force transmission point during the terminal stance phase is shifted in the anterior direction by stiffening in the forefoot region (42).

13. The prosthetic foot as claimed in one of the preceding claims, **characterized in that** the foot casing (4) has a stiffening in the midfoot region, said stiffening extending into the forefoot region (42).

14. The prosthetic foot as claimed in one of the preceding claims, **characterized in that** the receiving portion (6) and/or the core (2) have/has an interchangeable embodiment or an embodiment which is removable from the foot casing (4).

## Revendications

1. Prothèse de pied, comportant un coeur (2) sur lequel est agencé un moyen de fixation proximal (3) pour immobiliser la prothèse de pied (1) sur un tube de bas de jambe ou sur une emboîture de bas de jambe et qui est entouré par une enveloppe de pied (4) qui réalise une cavité (41) dans laquelle le coeur (2) est inséré,
dans lequel
une portion de réception (6) ayant un évidement (45) en forme de fente est réalisée dans une zone de pied avant (42) de l'enveloppe de pied (4), évidement dans lequel est introduite une languette (5) agencée sur le coeur (2),
le coeur (2) comprend un ressort à lame (25) ou est réalisé sous forme de ressort à lame, dont l'extrémité avant constitue la languette (5), et/ou
la languette (5) est fixée au coeur (2) et dépasse au-delà de l'extrémité avant du coeur (2),
**caractérisée en ce que**
la portion de réception (6) est réalisée sous forme de portion de réception séparée.

2. Prothèse de pied selon la revendication 1,
**caractérisée en ce que**
la portion de réception (6) remplit la zone de pied avant (42).

3. Prothèse de pied selon la revendication 2,
**caractérisée en ce que**
la zone de pied avant (42) et/ou la portion de réception (6) présentent une rigidité qui diffère de celle du reste de l'enveloppe de pied (4).

4. Prothèse de pied selon la revendication 2 ou 3,
**caractérisée en ce que**
la portion de réception (6) est insérée et/ou collée ou moulée dans l'enveloppe de pied (4).

5. Prothèse de pied selon l'une des revendications précédentes,
**caractérisée en ce que**
le coeur (2) est immobilisé par coopération de forme sur l'enveloppe de pied (4) dans une zone de talon (21).

6. Prothèse de pied selon l'une des revendications précédentes,
**caractérisée en ce que**
le coeur (2) comprend un rembourrage de talon (7).

7. Prothèse de pied selon l'une des revendications précédentes,
**caractérisée en ce que**
la languette (5) est réalisée sous forme de sangle ou de ressort à lame.

8. Prothèse de pied selon l'une des revendications précédentes,
**caractérisée en ce que**
une sangle ou un ressort à lame est intégré(e) dans la portion de réception (6) et/ou dans l'enveloppe de pied (4).

9. Prothèse de pied selon l'une des revendications précédentes,
**caractérisée en ce que**
la languette (5) est fixée à la face inférieure du coeur (2).

10. Prothèse de pied selon l'une des revendications précédentes,
**caractérisée en ce que**
la languette (5) pénètre jusque dans une zone d'orteils (43) de la prothèse de pied (1).

11. Prothèse de pied selon l'une des revendications précédentes,
**caractérisée en ce que**
dans la zone de pied avant (42), il est prévu un élément d'insert séparé (51) présentant une rigidité qui diffère de celle de l'enveloppe de pied (4) ou de la portion de réception (6).

12. Prothèse de pied selon l'une des revendications précédentes,
**caractérisée en ce que**
grâce à une rigidification dans la zone de pied avant (42), le point d'application de force pendant la phase debout terminale est déplacé en direction antérieure.

13. Prothèse de pied selon l'une des revendications précédentes,
**caractérisée en ce que**
l'enveloppe de pied (4) présente une rigidification dans la zone de métatarse, qui s'étend jusque dans la zone de pied avant (42).

14. Prothèse de pied selon l'une des revendications précédentes,
**caractérisée en ce que**
la portion de réception (6) et/ou le coeur (2) est réalisé(e) de façon interchangeable ou extractible hors de l'enveloppe de pied (4).
